# EUROPEAN PATENT SPECIFICATION

(11) **EP 4 296 263 B1**
(45) Date of publication and mention of the grant of the patent: **21.10.2026**
(21) Application number: 23153189.8
(22) Date of filing: 25.01.2023
(51) Int. Cl.: C07C 303/44, C07C 309/14

(54) **HIGH-EFFICIENCY CYCLIC PREPARATION METHOD FOR COLUMNAR TAURINE**
HOCHEFFIZIENTES ZYKLISCHES HERSTELLUNGSVERFAHREN FÜR SÄULENTAURIN
PROCÉDÉ DE PRÉPARATION CYCLIQUE À HAUT RENDEMENT POUR TAURINE EN COLONNE

(30) Priority: 22.06.2022 CN 202210720762
(43) Date of publication of application: 27.12.2023
(73) Proprietor: Qiangjiang Yongan Pharmaceutical Co., Ltd., Qianjiang, Hubei 433100 (CN)
(72) Inventor: Chen, Yong, Qianjiang, Hubei, 433100 (CN); Fang, Xiquan, Qianjiang, Hubei, 433100 (CN); Li, Shaobo, Qianjiang, Hubei, 433100 (CN)
(74) Representative: Gulde & Partner

(56) References cited:
- CN-A- 101 671 283
- CN-A- 112 898 187

## Description

### Field

The present disclosure relates to the technical field of chemical medicine crystallization, and in particular to a high-efficiency cyclic preparation method for columnar taurine.

### Background

Taurine, with a chemical name of 2-aminoethanesulfonic acid, is a naturally occurring β-sulfonated amino acid, which is a sulfur-containing amino acid essential for human and mammal body activities. Although taurine is not directly involved in the synthesis of proteins and enzymes in vivo, it has extremely important physiological functions. It is not only involved in maintaining normal homeostasis within the body, but also has an important regulatory effect on the normal exertion of physiological functions of the systems, such as a central nervous system, a cardiovascular system, a digestive system, a urinary system, an immune system, an endocrine system, and a reproductive system.

Taurine has a wide range of applications due to unique physiological and pharmacological functions. Taurine added to foods can play a role in strengthening people's physique, preventing diseases, eliminating fatigue, improving work efficiency, and the like. When taurine is applied in the medicine field, it may contribute to treatment of diseases such as fatty liver, myocarditis, heart failure, and atherosclerosis. Taurine added to an animal feed can promote the growth, development and reproduction of animals. Taurine has a good market prospect.

At present, the industrial production methods of taurine in China mainly include an ethanolamine method and an ethylene oxide method, in which crystallization is a critical step during separation, purification and product refining. Taurine is a colorless or white, small needle-like crystal or crystalline powder, and is odorless and easily soluble in water. Currently, commercially available taurine products have problems of smaller crystal particle size, uneven distribution, low bulk density, poor fluidity, easy caking and the like. Further, there are disadvantages of low production efficiency, high raw material cost, large energy consumption of public works, low equipment utilization rate and the like due to a single form of crystallization method and complex process in production enterprises. The poor quality and low production efficiency of taurine crystals is one of the significant reasons that affect product price, enterprise benefit and market application.

Both of the patent CN101904819A and the patent CN103830186A describe a process involving the preparation of taurine granules by a wet mixing granulator. The process is simple, but has strict requirements on the form and specific parameters of the granulator, large dependence on equipment, serious equipment loss, low utilization rate and high equipment cost.

The patent CN101857558A discloses a fractional cooling crystallization method, which is used to refine 2-aminoethanesulfonic acid from industrially pure 2-aminoethanesulfonic acid, avoiding the problem of caking of 2-aminoethanesulfonic acid. However, the method needs to be carried out under high-pressure reaction conditions. Meanwhile, the method also requires the addition of alkanol to promote decoloration and impurity removal with activated carbon, in combination with manners, such as a holding time of cooling crystallization of a filtrate, to obtain a taurine crystal. Not only will alkanol impurities remain in the taurine crystal, but also a crystal form of the obtained taurine crystal is cylindrical needle-like, which is also different from a columnar crystal form. In addition, the cylindrical needle-like taurine crystal further requires to be packaged in combination with an aluminum-plastic film to achieve a better purpose of preventing moisture absorption and caking of taurine.

The patent CN1872029A describes a process for preparing a spherical granular taurine product by controlling a dryer and a drying condition. The process is primitive, so that the energy consumption of public works is great, and the production cost is increased.

The patent CN101671283A describes a process for preparing columnar taurine crystals, by adding an alkaline substance into an aqueous taurine solution to adjust pH and then performing cooling crystallization. In the process, organic impurities are also introduced, increasing processing costs of raw materials and subsequent mother liquid.

The patent CN103848763A describes a process for changing the crystal morphology of taurine by using an organic carboxylate additive to obtain a columnar crystal product. The cost of raw materials in the process is low, and the obtained crystal form is also perfect. However, a single form of crystallization mode results in low yield in a once-through process. In order to improve the overall yield of crystallization operation, it is necessary to apply recycling of mother liquid for many times in industrial production, resulting in a cumbersome operation flow and a reduced production efficiency.

The patent CN109694336A describes a rhombohedral taurine crystal and a preparation process thereof, wherein a rhombohedral taurine crystal product is obtained. The process is simple but requires the addition of a certain amount of acid, in particular an organic acid, so that new impurities are introduced. Meanwhile, the acid may be enriched in industrial production, thereby increasing the subsequent processing difficulty of mother liquid after extraction of taurine crystals, and increasing the production cost, which is not convenient for the implementation of industrial mass production.

The patent CN109574886A describes a non-caking taurine crystal having an increased bulk density and fluidity, and a process for preparing the same. In the process, a certain amount of anti-solvent needed to be added to a system at a certain dropping rate, which introduces a large amount of organic impurities. The organic impurities may be enriched in industrial production, so that it is difficult for a mother liquid to separate completely in a subsequent process, and meanwhile, significant costs also need to be added for the separation.

The patent CN112479944A describes a taurine and a recrystallization method thereof, wherein a columnar crystal form is finally obtained by segmented control of cooling and heat preservation. The crystal has an axial length of 600µm-950µm, a radial length of 250µm-450µm, and a length-to-diameter ratio thereof in the axial direction to the radial direction is 2:1-3:1. Its particles are relatively small, and a micrograph of the crystal shows that the columnar crystal form thereof is somewhat different from a standard columnar crystal form, and that surfaces of the particles are irregular, which may affect the caking performance of the product.

CN 112 898 187 A describes a crystallization method of taurine comprising the steps of preparing an aqueous solution of taurine, treating the aqueous taurine solution with an alkaline resin to obtain a refined liquid, collecting the refined liquid and crystallizing taurine from the refined liquid.

CN 101 671 283 A discloses a crystallization method for preparing columnar taurine comprising the addition of an alkaline substance to a taurine solution.

### Summary

The invention is defined by the appended claims. The description that follows is subjected to this limitation. Any disclosure lying outside the scope of said claims is only intended for illustrative as well as comparative purposes. The present disclosure aims to provide a high-efficiency cyclic preparation method for a columnar taurine finished product, for solving at least one of the problems of existing taurine products, such as small crystal particle size, uneven distribution, low bulk density, poor fluidity, easy caking and the like.

The high-efficiency cyclic preparation method for the columnar taurine finished product includes: subjecting ethylene oxide and a bisulfite solution to an addition reaction to obtain an isethionate; mixing the resulting isethionate with ammonia for an ammonolysis reaction to obtain an ammonolysis reaction solution; evaporating the ammonolysis reaction solution to remove excess ammonia, so as to obtain a taurate; evaporating the ammonolysis reaction solution to remove excess ammonia, so as to obtain a taurate; converting the resulting taurate into a taurine solution; concentrating and crystallizing the taurine solution, and separating to obtain a taurine crude product and a taurine mother liquid; preparing an aqueous taurine solution with a certain concentration from the taurine crude product, and recycling the taurine mother liquid; introducing the aqueous taurine solution into a basic resin for treatment to remove an alkali metal isethionate and an isethionic acid derivative; monitoring an isethionate radical of an effluent liquid at an outlet of the basic resin, and qualifying the effluent liquid if a content of the isethionate radical in the effluent liquid is less than 0.05%; collecting the effluent liquid that is monitored to be qualified, and recycling the effluent liquid that is monitored to be unqualified; and crystallizing taurine of the collected effluent liquid to obtain a columnar taurine crystal.

According to one embodiment of the high-efficiency cyclic preparation method for the columnar taurine finished product, after a taurine crude product is mixed with water or a taurine mother liquid to be dissolved, insoluble solid impurities are removed to obtain the aqueous taurine solution.

According to one embodiment of the high-efficiency cyclic preparation method for the columnar taurine finished product, the process of preparing the aqueous taurine solution includes: dissolving, crystallizing and purifying the taurine crude product with water or a recycled refined taurine mother liquid, and then separating to obtain primary refined taurine and a primary refined taurine mother liquid; adding water or the recycled refined taurine mother liquid to the primary refined taurine, decolorizing and filtering, and performing cooling crystallization; carrying out solid-liquid separation on the taurine material after cooling crystallization, drying the separated refined wet taurine to obtain the taurine finished product, recovering a refined taurine mother liquid obtained after the solid-liquid separation, and preparing the aqueous taurine solution from the recovered refined taurine mother liquid.

According to one embodiment of the high-efficiency cyclic preparation method for the columnar taurine finished product, the basic resin column is regenerated with a base after a basic resin column is saturated.

According to one embodiment of the high-efficiency cyclic preparation method for the columnar taurine finished product, the method further includes: carrying out solid-liquid separation on the crystallized taurine material to obtain a refined taurine mother liquid; wherein the refined taurine mother liquid is mixed with a taurine crude product to be introduced into the basic resin as the aqueous taurine solution for treatment, so as to perform recycling; or the refined taurine mother liquid is mixed with the effluent liquid prior to the crystallizing for recycling.

According to one embodiment of the high-efficiency cyclic preparation method for the columnar taurine finished product, the cooling crystallization is performed by a crystallization kettle, and an effluent liquid is cooled to 5-35°C, preferably 15-25°C.

According to one embodiment of the high-efficiency cyclic preparation method for the columnar taurine finished product, a mass-volume concentration of the aqueous taurine solution is 5%-25%, preferably 10%-20%.

According to one embodiment of the high-efficiency cyclic preparation method for the columnar taurine finished product, the basic resin is a weakly basic resin, a strongly basic resin, or a combination of the two.

According to one embodiment of the high-efficiency cyclic preparation method for the columnar taurine finished product, a flow rate of allowing the aqueous taurine solution to pass through the basic resin is 0.25-25BV/h, preferably 5-15BV/h.

According to one embodiment of the high-efficiency cyclic preparation method for the columnar taurine finished product, the base used for regeneration of the basic resin is an alkali metal hydroxide, an alkali metal carbonate, an ammonia solution, or a combination thereof, wherein the base is preferably sodium hydroxide, an ammonia solution, or a combination of the two.

According to one embodiment of the high-efficiency cyclic preparation method for the columnar taurine finished product, an alkali metal isethionate and an isethionic acid derivative in an effluent liquid obtained after the basic resin is regenerated are recycled to an ammonolysis reaction.

According to one embodiment of the high-efficiency cyclic preparation method for the columnar taurine finished product, the effluent liquid that is monitored to be qualified is concentrated to a mass-volume ratio of 25%-40%, preferably a mass-volume ratio of 30%-35%, and then crystallized.

According to one embodiment of the high-efficiency cyclic preparation method for the columnar taurine finished product, the filtering is carried out by an organic nanofiltration membrane, a ceramic nanofiltration membrane, a SiC nanofiltration membrane, a metal microporous filter or a PE microporous filter, preferably by the SiC nanofiltration membrane.

According to one embodiment of the high-efficiency cyclic preparation method for the columnar taurine finished product, the crystallization is carried out in a crystallization kettle at a temperature that is controlled to be 60-80°C, preferably 65-75°C; and the cooling process is: an initial temperature is kept at 65-75°C for 0.5h-1.0h, and cooling is carried out at a cooling rate of 1°C-20°C/h.

According to one embodiment of the high-efficiency cyclic preparation method for the columnar taurine finished product, the solid-liquid separation is carried out by a plate centrifuge, a top-suspended centrifuge, a horizontal spiral centrifuge, a filter or a plate frame.

According to one embodiment of the high-efficiency cyclic preparation method for the columnar taurine finished product, the aqueous taurine crude product solution is prepared at a temperature of 0-100°C, preferably 35-80°C.

According to one embodiment of the high-efficiency cyclic preparation method for the columnar taurine finished product, the isethionate radical in the effluent liquid is monitored, and if the content of the isethionate radical is greater than 0.005%, the effluent liquid is unqualified; and preferably, if the content of the isethionate radical is greater than 0.001%, the effluent liquid is unqualified.

According to one embodiment of the high-efficiency cyclic preparation method for the columnar taurine finished product, the alkali metal isethionate is alkali metal sodium isethionate, alkali metal potassium isethionate or alkali metal lithium isethionate.

The present disclosure develops an improved high-efficiency cyclic preparation method for a columnar taurine crystal product aiming at the problems of small crystal particle size and uneven distribution, low bulk density, poor fluidity, easy caking and the like in the existing commercial taurine products, as well as the defects of low production efficiency, high raw material cost, large energy consumption of public works, low equipment utilization rate and the like due to a single crystallization mode and procedure in production enterprises. The method has the advantages of process innovation, high efficiency, and low production cost and energy consumption due to recycling, and the obtained crystal has the advantages of good quality, good fluidity, no caking, low production cost and high efficiency, and has good prospects for industrial production.

### Brief Description of the Drawings

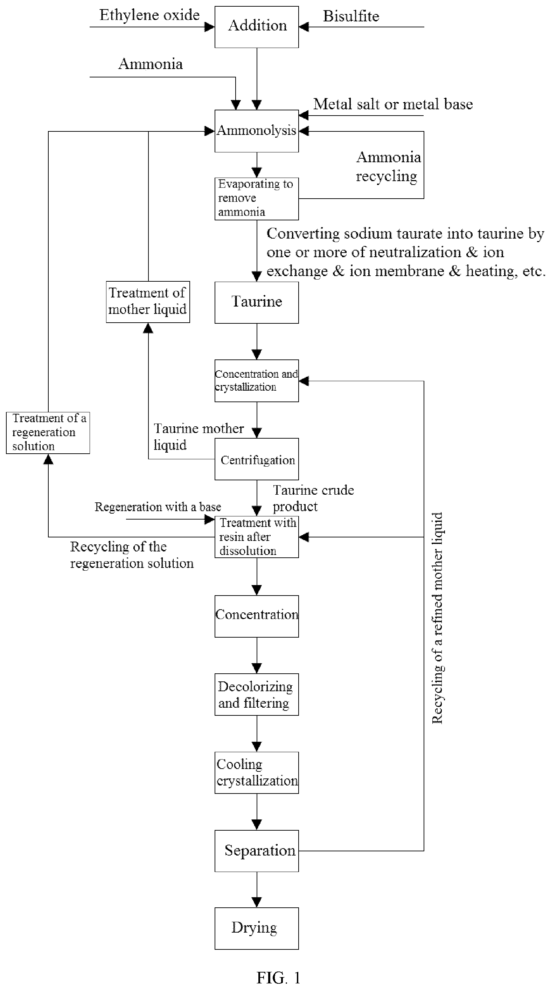

FIG. 1 is a flow chart of one embodiment of a cyclic preparation method for columnar taurine according to the present disclosure.

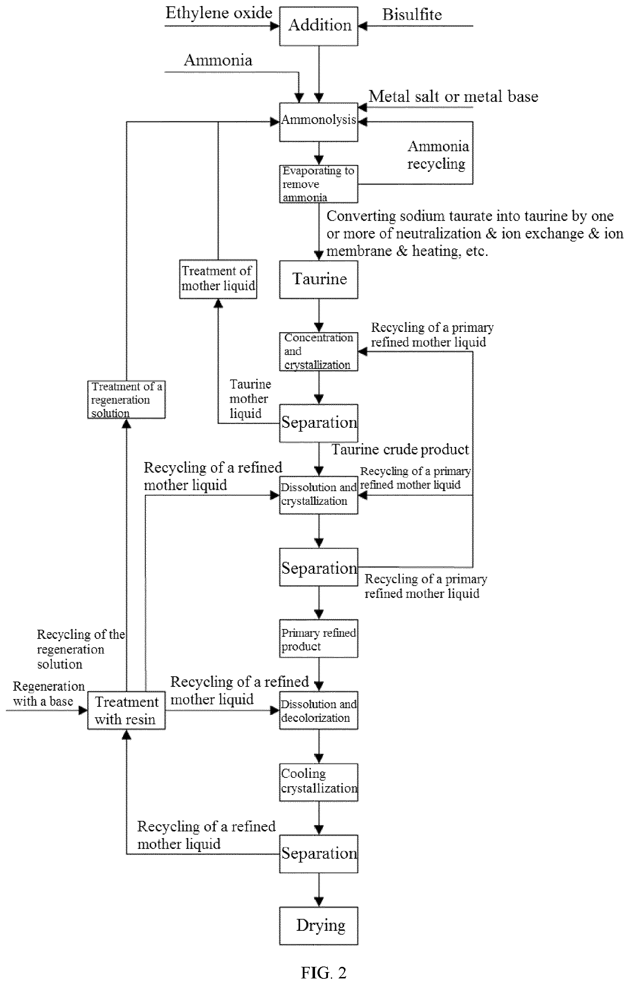

FIG. 2 is a flow chart of another embodiment of a cyclic preparation method for columnar taurine according to the present disclosure.

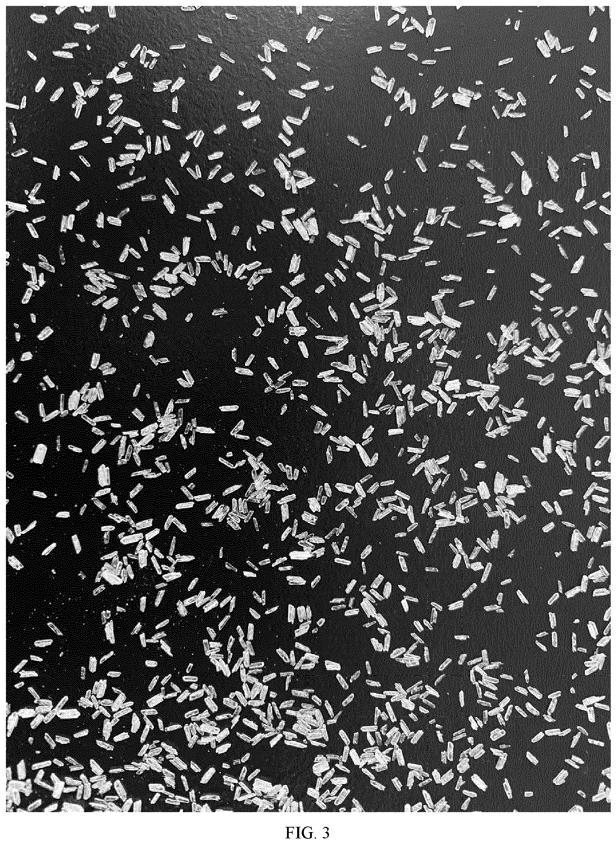

FIG. 3 is a crystal picture of a cyclic preparation method for columnar taurine according to the present disclosure.

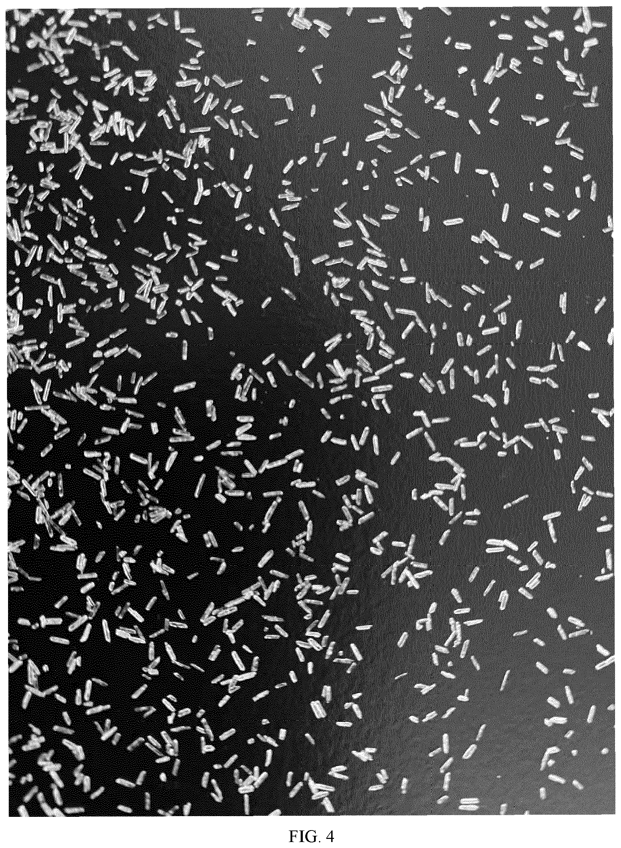

FIG. 4 is another crystal picture of a cyclic preparation method for columnar taurine according to the present disclosure.

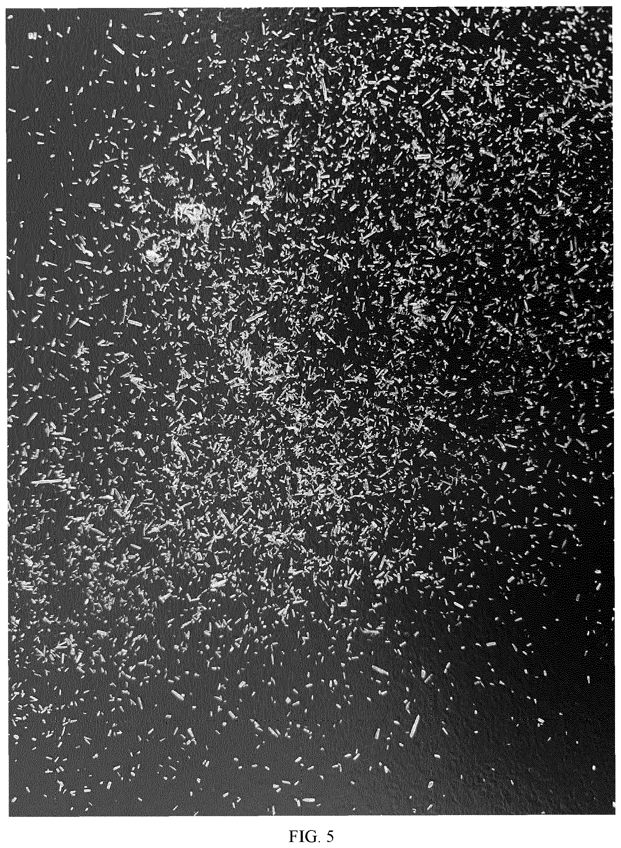

FIG. 5 is a crystal picture of taurine produced in the prior art.

### Detailed Description of the Embodiments

A cyclic preparation method for columnar taurine provided by the present disclosure will be further described below.

After long-term and intensive study, the inventors found that a taurine crude product produced by the existing taurine production process has a moisture content of about 8%. Therefore, the crude product also includes certain trace impurities, such as sodium isethionate, an isethionic acid derivative, a taurine derivative, ethylene glycol, polyether, ethanolamine, and sodium sulfate, thereby the above related impurities are contained in recrystallization of taurine. According to the principle of crystallography, factors influencing a crystal shape include change of supersaturation, change of a solvent system, and impurities; factors influencing the crystal purity include impurities in a mother liquid, a crystallization rate, a crystal particle size and particle size distribution; and factors influencing a crystal size include temperature, crystal nucleus quality, stirring, and the like. In combination with a taurine process for analysis, both of sodium isethionate and the isethionic acid derivative contain hydroxyl, which easily form hydrogen bonds with a taurine molecule, thereby affecting the structure of taurine crystals and thus having a certain effect on a growth process of the taurine crystals after nucleation. Meanwhile, since the solubility of sodium isethionate and the solubility of the isethionic acid derivative are much higher than that of taurine, after taurine is precipitated by cooling crystallization, the amount of taurine in the uncrystallized solution is reduced, while the sodium isethionate and the isethionic acid derivative are not affected due to the high solubility thereof, so that components in the solution are continuously changed during the cooling process, that is, the content of impurities is gradually increased, and the impurities strengthen the hydrogen bonding between the taurine crystal molecules, resulting in the formation of needle-like taurine. Therefore, the separation of the sodium isethionate and the isethionic acid derivative before crystallization causes a very low content of impurities in crystallization liquid, ensures the stability of the crystallization process, and realizes uniform columnar crystals of taurine. Furthermore, the separated isethionate can be used as a raw material for an ammonolysis reaction, thereby realizing recycling. After significant experiments and analyses by the inventors, it is found that an acid corresponding to the trace impurities sodium isethionate and the isethionic acid derivative contained in the taurine crude product is isethionic acid, which has a greater acidity than taurine. By using this property, a suitable resin may be employed to preferentially adsorb and separate impurities in the taurine crude product. A basic resin mainly adsorbs anions, such as an isethionate radical, a sulfate radical, a sulfite radical, and a chloride ion, and also adsorbs trace non-ionic impurities. After passing through the basic resin in the present disclosure, impurities, such as the isethionate radical, an isethionic acid derivative anion, the sulfate radical, and the sulfite radical, are mainly removed. Certainly, the adsorbed impurities may be eluted by a further resin regeneration step, and the effluent of the resin is collected. The adsorbed anions, such as the isethionate radical, the isethionic acid derivative anion, the sulfate radical, and the sulfite radical, may be desorbed upon regeneration of the resin, wherein ineffective ingredients such as the sulfate radical may be subsequently treated. According to the present disclosure, the separated solution is subjected to crystallization treatment, and the resin after adsorption is regenerated by a base to obtain an isethionate, and the obtained isethionate is used as a raw material for the ammonolysis reaction after treatment, thereby realizing cyclic production.

Accordingly, the present disclosure provides a cyclic preparation method for columnar taurine, which may specifically include:
Step S1. subjecting ethylene oxide and a bisulfite solution to an addition reaction to obtain an isethionate;
Step S2. mixing the isethionate obtained in S1 with ammonia under a condition of a metal salt or a metal base for an ammonolysis reaction to obtain an ammonolysis reaction solution;
Step S3. evaporating to remove excess ammonia after the ammonolysis reaction, so as to obtain a taurate;
Step S4. converting the resulting taurate into taurine;
Step S5. concentrating and crystallizing a taurine solution, separating to obtain a taurine crude product and a taurine mother liquid, wherein the taurine mother liquid may be returned to the step S2 after impurity removal treatment, as one of raw materials in S2;
S6. mixing the taurine crude product with water to be dissolved, removing insoluble solid impurities, and then passing through a basic resin column, collecting a taurine solution at an outlet of the basic resin column, and monitoring pH at the outlet and an isethionate radical in the taurine solution at the outlet, wherein if the taurine solution is unqualified, the collection is stopped, and the unqualified material may be returned to be used as raw materials for the steps in taurine production; and the basic resin column is regenerated with a base after the basic resin column is saturated, and an isethionate obtained after regeneration can be used as a raw material in the step S2 for recycling;
S7. concentrating, decolorizing, filtering the taurine solution at the outlet of the basic resin column, and then transferring the resulting solution to a crystallization kettle for cooling crystallization; and
S8. carrying out solid-liquid separation on the taurine material after cooling crystallization, drying the separated refined taurine wet product to obtain a taurine finished product, and returning the separated taurine refined mother liquid to the step S6 to be mixed with the taurine crude product, wherein the returned refined taurine mother liquid can replace water to a certain extent, without additionally adding water to mix with the taurine crude product; the refined taurine mother liquid may also be returned to the step S5, so as to be mixed with the taurine solution before or after the concentration and crystallization for recycling, preferably, the refined taurine mother liquid is returned to the step S6.

For some preferred embodiments, wherein
In the step S1, the bisulfite solution may be a sodium bisulfite solution having a concentration of 9-36wt%, wherein a molar ratio of sodium bisulfite to ethylene oxide is 1:0.95-1. The addition reaction may be carried out at 50-80°C under a pressure (expressed as an absolute pressure) of greater than 0.1Mpa.

In the step S2, the metal salt or metal base is any one or a mixture of any two or more of sodium hydroxide, potassium hydroxide, lithium hydroxide, sodium carbonate, potassium carbonate, and lithium carbonate. The ammonolysis reaction is carried out at 150-280°C under a pressure of 1-25MPa.

In the step S3, the evaporating to remove ammonia includes flash evaporation, single-effect or multi-effect evaporation, and the like, preferably flash evaporation.

In the step S3, the excess ammonia removed by evaporation may be returned to the step S2.

In the step S4, the taurate is converted into the taurine by acidification, ion exchange, an ionic membrane or heating, or the like, wherein an acid used in the acidification is sulfuric acid, hydrochloric acid, phosphoric acid, isethionic acid, sulfurous acid, or the like. Preferably, an ion exchange resin and the ionic membrane are adopted, and most preferably, the ion exchange resin is adopted.

In the step S6, the taurine crude product needs to be completely dissolved when mixed with water, wherein the temperature of a mixed solution of the taurine crude product and water is 0°C-100°C, preferably 35°C-80°C.

In the step S6, a mass-volume concentration of the aqueous taurine crude product solution is 5-25%, preferably 10-20%.

In the step S6, the basic resin is one of a weakly basic resin and a strongly basic resin, or a combination of the two, while the basic resin used does not adsorb taurine or has weak adsorption to taurine. Because the use of the basic resin is more effective for the treatment of anions in a solution.

In the step S6, the basic resin is one of a weakly basic resin and a strongly basic resin, or a combination of the two, while the resin used has a strong adsorption capacity for the isethionic acid and its derivatives.

In the step S6, a flow rate of allowing the aqueous taurine crude product solution to pass through the basic resin is controlled to be 0.25-25BV/h, and preferably, the flow rate is controlled to be 5-15BV/h.

In the step S6, the pH at the outlet of the basic resin column is monitored.

In the step S6, the isethionate radical is monitored. When the content of the isethionate radical in a solution collected by the basic resin is less than 0.05%, preferably less than 0.005%, more preferably less than 0.001%, the solution is qualified.

In the step S6, a basic solution used for the regeneration of the basic resin is an alkali metal hydroxide, an alkali metal carbonate, or an ammonia solution, preferably sodium hydroxide and the ammonia solution.

In the step S6, the isethionate obtained after regeneration of the basic resin can be returned to the step S2 directly or after treatment by concentrating, impurities removal and the like for an ammonolysis reaction to further synthesize the taurate.

In the step S6, the removed insoluble solid impurities may be selectively returned to each step for reuse, depending on the properties of the solid impurities.

In the step S6, when the basic resin column is regenerated with the base after the basic resin column is saturated, the adsorbed anions, such as the isethionate radical, the isethionic acid derivative anion, the sulfate radical, the sulfite radical, and the like, are desorbed. The ineffective ingredients, such as the sulfate radical, and the sulfite radical, are subsequently subjected to impurity removal treatment. A method for the impurity removal treatment specifically includes, for example, concentration to remove impurities, cooling to remove impurities, decoloration to remove impurities, and introducing ammonia to remove impurities. Certainly, the impurities obtained after the impurity removal treatment may be recovered.

In the step S7, the taurine solution is concentrated to a mass-volume ratio of 25%-40%, preferably 30%-35%.

In the step S7, an adsorbent used for the decolorizing includes activated carbon, a metal or non-metal oxide adsorbent (e.g., silica gel, alumina, a molecular sieve, natural clay, etc.), a polymer adsorbent, and the like. The mass of the adsorbent is 0.01%-0.1%, preferably 0.04%-0.08%, of the mass of the taurine crude product in the step S6.

In the step S7, a filtering mode includes an organic nanofiltration membrane, a ceramic nanofiltration membrane, a SiC nanofiltration membrane, a metal microporous filter, a PE microporous filter, and the like, preferably the SiC nanofiltration membrane.

In the step S7, a crystallization temperature of crystal from crystallization liquid in a crystallization kettle is controlled to be 60-80°C, preferably 65-75°C.

In the step S7, during the process of the cooling crystallization, a heat preservation stage is set around the crystal precipitation temperature, so that crystal particles are larger. The cooling process in the crystallization kettle is performed as follows: first heat preservation is performed at 65-75°C for 0.2h-1.0h, and cooling is performed at a cooling rate of 1°C-20°C/h.

In the step S8, a device used for the solid-liquid separation may be a centrifuge, a filter, a plate frame, or the like, preferably a plate centrifuge, a top-suspended centrifuge, and a horizontal spiral centrifuge.

For still another embodiment of the high-efficiency cyclic preparation method for the columnar taurine according to the present disclosure, the taurine crude product can be first purified to obtain a primary refined product. After the content of the sodium isethionate and the content of the isethionic acid derivative are reduced, the basic resin can be used to treat the recycled refined mother liquid, thereby cyclically preparing the columnar taurine, which may specifically include:
S1. subjecting ethylene oxide and a bisulfite solution to an addition reaction to obtain an isethionate;
S2. mixing the isethionate obtained in S1 with ammonia under a condition of a metal salt or a metal base for an ammonolysis reaction to obtain an ammonolysis reaction solution;
S3. evaporating to remove excess ammonia after the ammonolysis reaction, so as to obtain a taurate;
S4. converting the resulting taurate into taurine;
S5. concentrating and crystallizing a taurine solution, separating to obtain a taurine crude product and a taurine mother liquid, wherein the taurine mother liquid can be treated and then returned to the step S2;
S6. dissolving, crystallizing and purifying the taurine crude product in S5 with water or the refined taurine mother liquid, separating to obtain primary refined taurine and a primary refined taurine mother liquid, and returning the separated primary refined taurine mother liquid to S5 for treatment or recycling the separated primary refined taurine mother liquid to S6 to mix with the taurine crude product again;
S7. adding water or the refined taurine mother liquid (which may be a refined mother liquid after treatment in step S9), and activated carbon into the primary refined taurine, stirring and then heating for decolorizing, filtering, and inputting the filtered solution to a crystallization kettle for cooling crystallization;
S8. carrying out solid-liquid separation on the taurine material after cooling crystallization, and drying the separated refined wet taurine to obtain a taurine finished product; and
S9. allowing the refined taurine mother liquid separated in the step S8 to pass through a basic resin column, and collecting a taurine solution at an outlet of the basic resin column, and monitoring pH at the outlet and an isethionate radical in the taurine solution at the outlet, wherein the basic resin column is regenerated with a base after the basic resin column is saturated, and an isethionate obtained after regeneration can be used as a raw material in the step S2 for recycling; the collected taurine solution, namely the refined taurine mother liquid, is returned to S7 for recycling; the refined taurine mother liquid may also be returned to S6 to be mixed with the taurine crude product; and the refined taurine mother liquid may replace water to a certain extent, thereby reducing additional water addition.

Hereinafter, the relevant effects of the cyclic preparation method for the columnar taurine are further explained by a number of specific embodiments. The following embodiments show the practice of the present disclosure. The raw materials used are commercially available products unless otherwise specified, and the methods used are conventional methods unless otherwise specified. Unless otherwise specified, the material content refers to a mass-volume percent. The content of substances in a detection reaction can be displayed by HPLC and LC-MS analysis methods.

For one embodiment, a process for preparing a crude taurine product includes:
(1) preparation of sodium isethionate: a certain amount of sodium bisulfite solution was reacted with ethylene oxide in a molar ratio of sodium bisulfite to ethylene oxide being 1:0.95-1:1 under a pressure of 0.1Mpa at a pH of 5.0-9.0 at a temperature of 60-80°C to obtain sodium isethionate.
(2) Preparation of sodium taurate: sodium isethionate was subjected to an ammonolysis reaction with ammonia under a condition of adding sodium hydroxide. The ammonolysis reaction was carried out at a temperature of 250-270°C under a pressure of 10-15MPa for 45 min. After completion of the reaction, ammonia gas was removed by flash evaporation to obtain a sodium taurate solution.
(3) Conversion of sodium taurate into taurine: the sodium taurate solution in the step (2) was taken, and allowed to pass through an acidic resin column at a certain flow rate, and a material solution, namely a taurine solution, at an outlet of the resin column was collected. When indexes of the material solution at the outlet were substantially consistent with indexes of a feeding solution, for example, pH of the material solution at the outlet is close to pH of the feeding solution, feeding and discharging were stopped. The material solution in the resin column was washed by using deionized water and recovered. After the material was washed, an acid was allowed to pass through the resin at a certain flow rate for regeneration. After the regeneration, the resin was washed with pure water, and then ready for next feeding.
(4) Preparation of a taurine crude product: about 1850mL of the collected taurine solution was taken, with the taurine content of 6.7% detected in the material, concentrated, cooled to 20°C for crystallization, and separated by centrifugation to obtain 125g of the taurine crude product. The taurine crude product has a mass percentage of 92.5%, and a moisture content of 7%. A remaining taurine mother liquid was 72mL, and a mass-volume percent of the taurine mother liquid was 11.56%.

Based on the above-described process for the preparation of the taurine crude product, a process existing in the art for the extraction of taurine was employed, including as follows:

500g of the prepared taurine crude product (with a mass content of 92.5%, and a moisture content of 7%) was taken, and 1160g of water and 0.5 g of activated carbon were added. The mixture was stirred, heated to 90°C for 15 min, and then filtered while it was hot. The filtrate was poured into a crystallization flask, kept at 65-75°C for 30 min, followed by cooling to 20°C by controlling a cooling rate to be 10°C/h, and then separated and dried to obtain 324g of a refined dry taurine product and 1380mL of a refined taurine mother liquid (with the taurine content of 9.86%). It was detected that the content of an isethionate radical in the filtrate before the cooling crystallization was 0.28%. The obtained taurine was an elongated needle-like crystal of which a particle size was 0.1-1mm in length, and the obtained taurine had a bulk density of 0.7g/mL. The sieving results of particle sizes are specifically shown in Table 1:

**Table 1**

| Sieving data | | | | | | | | |
|---|---|---|---|---|---|---|---|---|
| Mesh | <40 | 40-60 | 60-80 | 80-100 | 100-120 | 120-150 | >150 | |
| Mass | 3.05 | 20.65 | 32.75 | 20.5 | 14.35 | 5.55 | 3.05 | 99.9 |
| Proportion | 3.05% | 20.67% | 32.78% | 20.52% | 14.36% | 5.56% | 3.05% | |

In comparison with the prior art described above, a method of extracting taurine in the method according to the present disclosure includes:

500g of the prepared taurine crude product (with a mass content of 92.5%, and a moisture content of 7%) was taken, and 2500g of water was added. The mixture was stirred, and heated to be dissolved, followed by prefiltration to remove insoluble impurities. The solution was allowed to pass through a resin column containing 125 ml of basic resin at a rate of 10 BV/h under a heat preservation condition. A solution at an outlet of the resin column was collected. When the pH of the solution was detected to be 7.5, the obtained solution was concentrated to a mass-volume ratio of greater than 30%. 0.1 g of activated carbon was added thereto. After the temperature was kept for 15 min, the solution was filtered while it was hot. The filtrate was poured into a crystallization flask, and kept at 65-75°C for 30 min, followed by cooling to 20°C by controlling a cooling rate to be 10°C/h, and then separated and dried to obtain 353g of a refined dry taurine product and 1125mL (with a content of 9.68%) of a refined taurine mother liquid. It was detected that the content of an isethionate radical in the taurine filtrate before the cooling crystallization was 0.02%. The obtained taurine was a columnar crystal of which a particle size was 2-4mm in length, 0.4-0.6mm in width and 0.2-0.4mm in thickness. The obtained taurine had a bulk density of 0.87g/mL. The sieving results of particle sizes are specifically shown in Table 2:

**Table 2**

| Sieving data | | | | | | | | |
|---|---|---|---|---|---|---|---|---|
| Mesh | <40 | 40-60 | 60-80 | 80-100 | 100-120 | 120-150 | >150 | |
| Mass | 45.8 | 42.3 | 9.7 | 1.1 | 0.6 | 0.3 | 0.1 | 99.9 |
| Proportion | 45.85% | 42.34% | 9.71% | 1.10% | 0.60% | 0.30% | 0.10% | |

Further, for one specific embodiment, a process for recycling a material (i.e., a regeneration solution) obtained after the columnar crystal was prepared, includes:
(1) collection of the regeneration solution: the basic resin that was saturated after adsorbing was regenerated with 600g of a sodium hydroxide solution having a mass percentage of 5%, and the solution after regeneration was collected.
(2) Returning for preparation of sodium taurate: the regeneration solution was mixed with a sodium isethionate solution, wherein a molar ratio of the regeneration solution in the mixed solution may account for 0.1%-100%. Sodium hydroxide and ammonia were added to perform an ammonolysis reaction at a temperature of 250-270°C under a pressure of 10-15MPa for 45 minutes. After the reaction was completed, ammonia gas was removed by flash evaporation, and the resulting solution was a sodium taurate solution. If the regeneration solution was obtained by regeneration using sodium hydroxide, and the amount of sodium hydroxide in the mixed solution to which the regeneration solution was added reached 0.1% or more of the molar amount of sodium isethionate in the mixed solution, sodium hydroxide may no longer be added before the ammonolysis. If the regeneration solution was obtained by regeneration using ammonia, sodium hydroxide needs to be added.
(3) Conversion of sodium taurate into taurine: the above sodium taurate solution was taken and allowed to pass through an acidic resin column at a certain flow rate, and a material solution, namely a taurine solution, at an outlet of the resin column was collected. When indexes of the material solution at the outlet were substantially consistent with indexes of a feeding solution (for example, approximate pH), feeding was stopped. The material solution in the resin column was washed by using deionized water and recovered. After the material was washed, an acid was allowed to pass through the resin at a certain flow rate for regeneration. After the regeneration, the resin was washed with pure water, and then ready for next feeding.
(4) Preparation of a taurine crude product: about 8000mL of the collected taurine solution was taken, with the taurine content of 6.8% detected, concentrated, cooled to 20°C for crystallization, and separated by centrifugation to obtain 550g of the crude product, wherein a mass percentage of the taurine was 92.6%, and a moisture content was 6.9%. A remaining mother liquid was 300mL, and a mass-volume percent of the taurine mother liquid was 11.45%.
(5) Crystallization of taurine: 500g of the resulting taurine crude product (with a mass content of 92.6%, and a moisture content of 6.9%) was taken, 2500g of water was added, the mixture was stirred and heated to be dissolved, and the insoluble impurities were removed by prefiltration. Then, the solution was allowed to pass through a 125ml basic resin column at a rate of 10BV/h under a heat preservation condition. A solution at an outlet of the resin column was collected, and a pH value thereof was detected to be 7.5. Then, the solution obtained at the outlet was concentrated to a mass-volume ratio of greater than 30%, and 0.1 g of activated carbon was added thereto. After the temperature was kept for 15 min, the solution was filtered while it was hot. The filtrate was poured into a crystallization flask, kept at 65-75°C for 30 min, followed by cooling to 20°C by controlling a cooling rate to be 10°C/h, and then separated and dried to obtain 354g of a refined dry product and 1115mL of a refined mother liquid (with a content of 9.65%). It was detected that the content of an isethionate radical in the filtrate before the cooling crystallization was 0.01%. The resulting taurine was a columnar crystal of which a particle size was 2-4mm in length, 0.4-0.6mm in width, and 0.2-0.4mm in thickness. The bulk density of the resulting taurine was 0.85g/mL. The sieving results of particle sizes are specifically shown in Table 3:

**Table 3**

| Sieving data | | | | | | | | |
|---|---|---|---|---|---|---|---|---|
| Mesh | <40 | 40-60 | 60-80 | 80-100 | 100-120 | 120-150 | >150 | |
| Mass | 42.6 | 43.3 | 10.8 | 2.2 | 0.5 | 0.4 | 0.1 | 99.9 |
| Proportion | 42.64% | 43.34% | 10.81% | 2.20% | 0.50% | 0.40% | 0.10% | |

Further, the crystallization of taurine was performed in the case where the taurine crude product was applied to the refined mother liquid, so as to verify whether the crystallization effect of taurine was deteriorated after the taurine crude product was applied to the refined mother liquid.

A specific taurine crystallization process included:
500g of the prepared taurine crude product (with a mass content of 92.6%, and a moisture content of 6.9%) was taken, and 1100ml of the refined mother liquid obtained in the crystallization of taurine was added, and 1900g of pure water was added. The mixture was stirred and heated to be dissolved, and the insoluble impurities were removed by prefiltration. Then, the solution was allowed to pass through a 125ml basic resin column at a rate of 10BV/h under a heat preservation condition. A solution at an outlet of the resin column was collected, and a pH value thereof was detected to be 7.5. Then, the obtained solution was concentrated to a mass-volume ratio of greater than 30%. 0.1 g of activated carbon was added thereto. The temperature was kept for 15 min, and then the solution was filtered while it was hot. The filtrate was poured into a crystallization flask, kept at 65-75°C for 30 min, followed by cooling to 20°C by controlling a cooling rate to be 10°C/h, and then separated and dried to obtain 440g of a refined dry product and 1350mL of a refined mother liquid (with a content of 9.70%). It was detected that the content of an isethionate radical in the filtrate before the cooling crystallization was 0.01%. The resulting taurine was a columnar crystal of which a particle size was 2-4mm in length, 0.4-0.6mm in width and 0.2-0.4mm in thickness. The resulting taurine had a bulk density of 0.86g/mL. The sieving results of particle sizes are specifically shown in Table 4:

**Table 4**

| Sieving data | | | | | | | | |
|---|---|---|---|---|---|---|---|---|
| Mesh | <40 | 40-60 | 60-80 | 80-100 | 100-120 | 120-150 | >150 | |
| Mass | 43.5 | 43.9 | 9.8 | 1.8 | 0.5 | 0.3 | 0.1 | 99.90 |
| Proportion | 43.54% | 43.94% | 9.81% | 1.80% | 0.50% | 0.30% | 0.10% | |

Further, by a control experiment group, the influence of artificial addition of sodium isethionate after the impurities were removed by the resin, on taurine crystallization during the crystallization of taurine was verified, showing that the sodium isethionate had a great influence on the crystallization.

A specific taurine crystallization process included:
500g of the resulting taurine crude product (with a mass content of 92.5%, and a moisture content of 7%) was taken and 2500g of water was added, the mixture was stirred and heated to be dissolved, and insoluble impurities was removed by prefiltration. Then, the solution was allowed to pass through a 125ml basic resin column at a rate of 10BV/h under a heat preservation condition. A solution at an outlet of the resin column was collected, and a pH value thereof was detected to be 7.5. Then, the solution obtained at the outlet was concentrated to a mass-volume ratio of greater than 30%. 0.1 g of activated carbon and 0.5 g of sodium isethionate were added thereto. The temperature was kept for 15 min, and then the solution was filtered while it was hot. The filtrate was poured into a crystallization flask, kept at 65-75°C for 30 min, followed by cooling to 20°C by controlling a cooling rate to be 10°C/h, and then separated and dried to obtain 335g of a refined dry product and 1300mL of a refined mother liquid (with a content of 9.75%). It was detected that the content of an isethionate radical in the filtrate before the cooling crystallization was 0.05%. The resulting taurine was an elongated needle-like crystal of which a particle size was 0.1-1 mm in length. The resulting taurine had a bulk density of 0.71g/mL. The sieving results of particle sizes are specifically shown in Table 5:

**Table 5**

| Sieving data | | | | | | | | |
|---|---|---|---|---|---|---|---|---|
| Mesh | <40 | 40-60 | 60-80 | 80-100 | 100-120 | 120-150 | >150 | |
| Mass | 4.15 | 26.75 | 31.35 | 16.5 | 14.35 | 4.65 | 2.15 | 99.9 |
| Proportion | 4.15% | 26.78% | 31.38% | 16.52% | 14.36% | 4.65% | 2.15% | |

As can be seen from the above embodiments, the taurine crystal obtained according to the present disclosure has a columnar crystal form, larger particles and uniform particle size distribution. Specifically, the particle size is 2-4mm in length, 0.4-0.6mm in width, and 0.2-0.4mm in thickness. The sieving of particle sizes is that: the percentage of 40-mesh or less is greater than 35%, the percentage of 80-mesh or less is greater than 90%, and the percentage of fine powder with 120-mesh or more is less than 3%. The resulting taurine has a high bulk density of 0.8g/mL-0.9g/mL. The overall fluidity is good, the filtration and drying speed is high, and the packaging, storage, transportation, use and other properties are excellent and the resulting taurine is more convenient to use. In addition, any auxiliary substance does not be added during the crystallization in the cyclic preparation method for the columnar taurine according to the present disclosure, so that there is no residue of any auxiliary substance in the taurine product. In addition, the crystallization liquid is purified, so that the purity of the taurine product is more ensured, and the mass content of taurine is greater than 99.8%. Meanwhile, the method of the present disclosure realizes recycling, greatly reduces the production cost, and is very convenient for industrial implementation. In addition, it should be noted that the crystallization process of taurine according to the present disclosure can be operated intermittently or continuously, and the crystallization process is carried out under normal pressure. The conditions are easily controlled, which is more favorable for industrial large-scale production.

In summary, based on the long-term experimental summary of the inventors, the present disclosure provides a method for preparing columnar taurine crystals, which can make taurine crystals have uniform particle size distribution, high bulk density, good fluidity, no caking, fast filtering and drying rate, and excellent packaging, storage, transportation, and use performance. Meanwhile, the recycling of the taurine mother liquid is realized. The problems of existing taurine products, such as an elongated crystal form, a small crystal form, and easy caking, are cleverly solved and no new organic substance or the like is introduced. The product has a very high purity, and the method is simple to operate, and is very convenient for industrial implementation.

## Claims

1. A high-efficiency cyclic preparation method for a columnar taurine finished product, comprising:
subjecting ethylene oxide and a bisulfite solution to an addition reaction to obtain an isethionate;
mixing the resulting isethionate with ammonia for an ammonolysis reaction to obtain an ammonolysis reaction solution;
evaporating the ammonolysis reaction solution to remove excess ammonia, so as to obtain a taurate;
converting the resulting taurate into a taurine solution;
concentrating and crystallizing the taurine solution, and separating to obtain a taurine crude product and a taurine mother liquid;
preparing an aqueous taurine solution with a certain concentration from the taurine crude product, and recycling the taurine mother liquid;
introducing the aqueous taurine solution into a basic resin for treatment to remove an alkali metal isethionate and an isethionic acid derivative;
monitoring an isethionate radical of an effluent liquid at an outlet of the basic resin, and qualifying the effluent liquid if a content of the isethionate radical in the effluent liquid is less than 0.05%;
collecting the effluent liquid that is monitored to be qualified, and recycling the effluent liquid that is monitored to be unqualified; and
crystallizing taurine of the collected effluent liquid to obtain a columnar taurine crystal.

2. The high-efficiency cyclic preparation method for the columnar taurine finished product according to claim 1, wherein after a taurine crude product is mixed with water or a taurine mother liquid to be dissolved, insoluble solid impurities are removed to obtain the aqueous taurine solution.

3. The high-efficiency cyclic preparation method for the columnar taurine finished product according to claim 1, wherein a process of preparing the aqueous taurine solution comprises:
dissolving, crystallizing and purifying a taurine crude product with water or a recycled refined taurine mother liquid, and then separating to obtain primary refined taurine and a primary refined taurine mother liquid;
adding water or the recycled refined taurine mother liquid to the primary refined taurine, decolorizing and filtering, and performing cooling crystallization; and
carrying out solid-liquid separation on the taurine material after cooling crystallization, drying the separated refined wet taurine to obtain the taurine finished product, recovering a refined taurine mother liquid obtained after the solid-liquid separation, and preparing the aqueous taurine solution from the recovered refined taurine mother liquid.

4. The high-efficiency cyclic preparation method for the columnar taurine finished product according to claim 1, wherein the basic resin column is regenerated with a base after a basic resin column is saturated.

5. The high-efficiency cyclic preparation method for the columnar taurine finished product according to claim 1, further comprising: carrying out solid-liquid separation on the crystallized taurine material to obtain a refined taurine mother liquid;
wherein the refined taurine mother liquid is mixed with a taurine crude product to be introduced into the basic resin as the aqueous taurine solution for treatment, so as to perform recycling; or
the refined taurine mother liquid is mixed with the effluent liquid prior to the crystallizing for recycling.

6. The high-efficiency cyclic preparation method for the columnar taurine finished product according to claim 3, wherein the cooling crystallization is performed by a crystallization kettle, and an effluent liquid is cooled to 5-35°C, preferably 15-25°C.

7. The high-efficiency cyclic preparation method for the columnar taurine finished product according to claim 1, wherein a mass-volume concentration of the aqueous taurine solution is 5%-25%, preferably 10%-20%.

8. The high-efficiency cyclic preparation method for the columnar taurine finished product according to claim 1, wherein the basic resin is a weakly basic resin, a strongly basic resin, or a combination of the two.

9. The high-efficiency cyclic preparation method for the columnar taurine finished product according to claim 1, wherein a flow rate of allowing the aqueous taurine solution to pass through the basic resin is 0.25-25BV/h, preferably 5-15BV/h.

10. The high-efficiency cyclic preparation method for the columnar taurine finished product according to claim 4, wherein the base used for regeneration of the basic resin is an alkali metal hydroxide, an alkali metal carbonate, an ammonia solution, or a combination thereof, wherein the base is preferably sodium hydroxide, an ammonia solution, or a combination of the two.

11. The high-efficiency cyclic preparation method for the columnar taurine finished product according to claim 4, wherein an alkali metal isethionate and an isethionic acid derivative in an effluent liquid obtained after the basic resin is regenerated are recycled to an ammonolysis reaction.

12. The high-efficiency cyclic preparation method for the columnar taurine finished product according to claim 1, wherein the effluent liquid that is monitored to be qualified is concentrated to a mass-volume ratio of 25%-40%, preferably a mass-volume ratio of 30%-35%, and then crystallized.

13. The high-efficiency cyclic preparation method for the columnar taurine finished product according to claim 3, wherein the filtering is carried out by an organic nanofiltration membrane, a ceramic nanofiltration membrane, a SiC nanofiltration membrane, a metal microporous filter or a PE microporous filter, preferably by the SiC nanofiltration membrane.

14. The high-efficiency cyclic preparation method for the columnar taurine finished product according to claim 3, wherein the crystallization is carried out in a crystallization kettle at a temperature that is controlled to be 60-80°C, preferably 65-75°C; and
the cooling process is: an initial temperature is kept at 65-75°C for 0.5h-1.0h, and cooling is carried out at a cooling rate of 1°C-20°C/h.

15. The high-efficiency cyclic preparation method for the columnar taurine finished product according to claim 3 or 5, wherein the solid-liquid separation is carried out by a plate centrifuge, a top-suspended centrifuge, a horizontal spiral centrifuge, a filter or a plate frame.

16. The high-efficiency cyclic preparation method for the columnar taurine finished product according to claim 1, wherein the aqueous taurine crude product solution is prepared at a temperature of 0-100°C, preferably 35-80°C.

17. The high-efficiency cyclic preparation method for the columnar taurine finished product according to claim 1, wherein if the content of the isethionate radical is greater than 0.005%, the effluent liquid is unqualified; and preferably, if the content of the isethionate radical is greater than 0.001%, the effluent liquid is unqualified.

18. The high-efficiency cyclic preparation method for the columnar taurine finished product according to claim 1, wherein the alkali metal isethionate is alkali metal sodium isethionate, alkali metal potassium isethionate or alkali metal lithium isethionate.

## Patentansprüche

1. Hocheffizientes zyklisches Herstellungsverfahren für ein Säulentaurin-Endprodukt, umfassend:
Unterwerfen von Ethylenoxid und einer Bisulfitlösung einer Additionsreaktion, um ein Isethionat zu erhalten;
Mischen des resultierenden Isethionats mit Ammoniak für eine Ammonolysereaktion, um eine Ammonolysereaktionslösung zu erhalten;
Eindampfen der Ammonolysereaktionslösung, um überschüssiges Ammoniak zu entfernen und so ein Taurat zu erhalten;
Umwandeln des resultierenden Taurats in eine Taurinlösung;
Konzentrieren und Kristallisieren der Taurinlösung und Trennen, um ein Taurin-Rohprodukt und eine Taurin-Mutterlauge zu erhalten;
Herstellen einer wässrigen Taurinlösung mit einer bestimmten Konzentration aus dem Taurin-Rohprodukt und Wiederverwerten der Taurin-Mutterlauge;
Einleiten der wässrigen Taurinlösung in ein basisches Harz zur Behandlung, um ein Alkalimetall-Isethionat und ein Isethionsäurederivat zu entfernen;
Überwachen eines Isethionatradikals einer Abflussflüssigkeit an einem Auslass des basischen Harzes und Qualifizieren der Abflussflüssigkeit, wenn der Gehalt des Isethionatradikals in der Abflussflüssigkeit weniger als 0,05% beträgt;
Sammeln der als qualifiziert überwachten Abflussflüssigkeit und Wiederverwerten der als unqualifiziert überwachten Abflussflüssigkeit; und
Kristallisieren von Taurin der gesammelten Abflussflüssigkeit, um einen Säulentaurin-Kristall zu erhalten.

2. Hocheffizientes zyklisches Herstellungsverfahren für das Säulentaurin-Endprodukt nach Anspruch 1, wobei, nachdem ein Taurin-Rohprodukt mit Wasser oder einer aufzulösenden Taurin-Mutterlauge gemischt wurde, unlösliche feste Verunreinigungen entfernt werden, um die wässrige Taurinlösung zu erhalten.

3. Hocheffizientes zyklisches Herstellungsverfahren für das Säulentaurin-Endprodukt nach Anspruch 1, wobei ein Verfahren zur Herstellung der wässrigen Taurinlösung Folgendes umfasst:
Auflösen, Kristallisieren und Reinigen eines Taurin-Rohprodukts mit Wasser oder einer wiederverwerteten, raffinierten Taurin-Mutterlauge und anschließendes Trennen, um primär raffiniertes Taurin und eine primär raffinierte Taurin-Mutterlauge zu erhalten;
Zugeben von Wasser oder der wiederverwerteten, raffinierten Taurin-Mutterlauge zum primär raffinierten Taurin, Entfärben und Filtrieren sowie Durchführen einer Kühlkristallisation; und
Durchführen einer Fest-Flüssig-Trennung an dem Taurinmaterial nach der Kühlkristallisation, Trocknen des abgetrennten raffinierten nassen Taurins, um das Taurin-Endprodukt zu erhalten, Rückgewinnen einer nach der Fest-Flüssig-Trennung erhaltenen raffinierten Taurin-Mutterlauge und Herstellen der wässrigen Taurinlösung aus der rückgewonnenen raffinierten Taurin-Mutterlauge.

4. Hocheffizientes zyklisches Herstellungsverfahren für das Säulentaurin-Endprodukt nach Anspruch 1, wobei die basische Harzsäule mit einer Base regeneriert wird, nachdem eine basische Harzsäule gesättigt ist.

5. Hocheffizientes zyklisches Herstellungsverfahren für das Säulentaurin-Endprodukt nach Anspruch 1, umfassend ferner: das Durchführen einer Fest-Flüssig-Trennung an dem kristallisierten Taurinmaterial, um eine raffinierte Taurin-Mutterlauge zu erhalten;
wobei die raffinierte Taurin-Mutterlauge mit einem Taurin-Rohprodukt gemischt wird, um als die wässrige Taurinlösung zur Behandlung in das basische Harz eingeleitet zu werden, um so eine Wiederverwertung durchzuführen; oder
die raffinierte Taurin-Mutterlauge vor der Kristallisation zur Wiederverwertung mit der Abflussflüssigkeit gemischt wird.

6. Hocheffizientes zyklisches Herstellungsverfahren für das Säulentaurin-Endprodukt nach Anspruch 3, wobei die Kühlkristallisation durch einen Kristallisationskessel durchgeführt wird und eine Abflussflüssigkeit auf 5-35°C, vorzugsweise 15-25°C, abgekühlt wird.

7. Hocheffizientes zyklisches Herstellungsverfahren für das Säulentaurin-Endprodukt nach Anspruch 1, wobei eine Masse-Volumen-Konzentration der wässrigen Taurinlösung 5%-25%, vorzugsweise 10%-20%, beträgt.

8. Hocheffizientes zyklisches Herstellungsverfahren für das Säulentaurin-Endprodukt nach Anspruch 1, wobei das basische Harz ein schwach basisches Harz, ein stark basisches Harz oder eine Kombination aus beiden ist.

9. Hocheffizientes zyklisches Herstellungsverfahren für das Säulentaurin-Endprodukt nach Anspruch 1, wobei die Durchflussrate, mit der die wässrige Taurinlösung durch das basische Harz geleitet wird, 0,25-25 BV/h, vorzugsweise 5-15 BV/h, beträgt.

10. Hocheffizientes zyklisches Herstellungsverfahren für das Säulentaurin-Endprodukt nach Anspruch 4, wobei die zur Regeneration des basischen Harzes verwendete Base ein Alkalimetallhydroxid, ein Alkalimetallcarbonat, eine Ammoniaklösung oder eine Kombination davon ist, wobei die Base vorzugsweise Natriumhydroxid, eine Ammoniaklösung oder eine Kombination aus beiden ist.

11. Hocheffizientes zyklisches Herstellungsverfahren für das Säulentaurin-Endprodukt nach Anspruch 4, wobei ein Alkalimetall-Isethionat und ein Isethionsäurederivat in einer nach der Regeneration des basischen Harzes erhaltenen Abflussflüssigkeit in eine Ammonolysereaktion zurückgeführt werden.

12. Hocheffizientes zyklisches Herstellungsverfahren für das Säulentaurin-Endprodukt nach Anspruch 1, wobei die als qualifiziert überwachte Abflussflüssigkeit auf ein Masse-Volumen-Verhältnis von 25%-40 %, vorzugsweise auf ein Masse-Volumen-Verhältnis von 30%-35%, konzentriert und anschließend kristallisiert wird.

13. Hocheffizientes zyklisches Herstellungsverfahren für das Säulentaurin-Endprodukt nach Anspruch 3, wobei das Filtrieren mittels einer organischen Nanofiltrationsmembran, einer keramischen Nanofiltrationsmembran, einer SiC-Nanofiltrationsmembran, eines mikroporösen Metallfilters oder eines mikroporösen PE-Filters, vorzugsweise mittels der SiC-Nanofiltrationsmembran, durchgeführt wird.

14. Hocheffizientes zyklisches Herstellungsverfahren für das Säulentaurin-Endprodukt nach Anspruch 3, wobei die Kristallisation in einem Kristallisationskessel bei einer Temperatur durchgeführt wird, die auf 60-80°C, vorzugsweise 65-75°C, geregelt wird; und
der Abkühlvorgang wie folgt abläuft: Eine Anfangstemperatur wird bei 65-75°C für 0,5 h-1,0 h gehalten und die Abkühlung erfolgt mit einer Abkühlgeschwindigkeit von 1°C-20°C/h.

15. Hocheffizientes zyklisches Herstellungsverfahren für das Säulentaurin-Endprodukt nach Anspruch 3 oder 5, wobei die Fest-Flüssig-Trennung mittels einer Plattenzentrifuge, einer oben aufgehängten Zentrifuge, einer horizontalen Spiralzentrifuge, eines Filters oder eines Plattenrahmens durchgeführt wird.

16. Hocheffizientes zyklisches Herstellungsverfahren für das Säulentaurin-Endprodukt nach Anspruch 1, wobei die wässrige Taurin-Rohproduktlösung bei einer Temperatur von 0-100°C, vorzugsweise 35-80°C, hergestellt wird.

17. Hocheffizientes zyklisches Herstellungsverfahren für das Säulentaurin-Endprodukt nach Anspruch 1, wobei die Abflussflüssigkeit unqualifiziert ist, wenn der Gehalt des Isethionatradikals größer als 0,005% ist; und vorzugsweise wenn der Gehalt des Isethionatradikals größer als 0,001% ist, die Abflussflüssigkeit unqualifiziert ist.

18. Hocheffizientes zyklisches Herstellungsverfahren für das Säulentaurin-Endprodukt nach Anspruch 1, wobei das Alkalimetall-Isethionat Alkalimetall-Natriumisethionat, Alkalimetall-Kaliumisethionat oder Alkalimetall-Lithiumisethionat ist.

## Revendications

1. Procédé de préparation cyclique à haut rendement d'un produit fini de taurine colonnaire, comprenant :
la soumission d'oxyde d'éthylène et d'une solution de bisulfite à une réaction d'addition afin d'obtenir un iséthionate ;
le mélange de l'iséthionate résultant avec de l'ammoniac pour une réaction d'ammonolyse afin d'obtenir une solution de réaction d'ammonolyse ;
l'évaporation de la solution de réaction d'ammonolyse pour éliminer l'ammoniac en excès, de manière à obtenir un taurate ;
la conversion du taurate résultant en une solution de taurine ;
la concentration et la cristallisation de la solution de taurine, et la séparation afin d'obtenir un produit brut de taurine et une liqueur mère de taurine ;
la préparation, à partir du produit brut de taurine, d'une solution aqueuse de taurine ayant une certaine concentration, et le recyclage de la liqueur mère de taurine ;
l'introduction de la solution aqueuse de taurine dans une résine basique pour un traitement destiné à éliminer un iséthionate de métal alcalin et un dérivé d'acide iséthionique ;
la surveillance d'un radical iséthionate d'un liquide effluent au niveau d'une sortie de la résine basique, et la qualification du liquide effluent si une teneur du radical iséthionate dans le liquide effluent est inférieure à 0,05 % ;
la collecte du liquide effluent surveillé comme étant qualifié, et le recyclage du liquide effluent surveillé comme étant non qualifié ; et
la cristallisation de la taurine du liquide effluent collecté afin d'obtenir un cristal de taurine colonnaire.

2. Procédé de préparation cyclique à haut rendement du produit fini de taurine colonnaire selon la revendication 1, dans lequel, après qu'un produit brut de taurine est mélangé avec de l'eau ou une liqueur mère de taurine afin d'être dissous, des impuretés solides insolubles sont éliminées afin d'obtenir la solution aqueuse de taurine.

3. Procédé de préparation cyclique à haut rendement du produit fini de taurine colonnaire selon la revendication 1, dans lequel un processus de préparation de la solution aqueuse de taurine comprend :
la dissolution, la cristallisation et la purification d'un produit brut de taurine avec de l'eau ou une liqueur mère de taurine raffinée recyclée, puis la séparation afin d'obtenir de la taurine raffinée primaire et une liqueur mère de taurine raffinée primaire ;
l'ajout d'eau ou de la liqueur mère de taurine raffinée recyclée à la taurine raffinée primaire, la décoloration et la filtration, et la réalisation d'une cristallisation par refroidissement ; et
la réalisation d'une séparation solide-liquide sur la matière de taurine après la cristallisation par refroidissement, le séchage de la taurine humide raffinée séparée afin d'obtenir le produit fini de taurine, la récupération d'une liqueur mère de taurine raffinée obtenue après la séparation solide-liquide, et la préparation de la solution aqueuse de taurine à partir de la liqueur mère de taurine raffinée récupérée.

4. Procédé de préparation cyclique à haut rendement du produit fini de taurine colonnaire selon la revendication 1, dans lequel la colonne de résine basique est régénérée avec une base après que la colonne de résine basique est saturée.

5. Procédé de préparation cyclique à haut rendement du produit fini de taurine colonnaire selon la revendication 1, comprenant en outre : la réalisation d'une séparation solide-liquide sur la matière de taurine cristallisée afin d'obtenir une liqueur mère de taurine raffinée ;
dans lequel la liqueur mère de taurine raffinée est mélangée avec un produit brut de taurine afin d'être introduite dans la résine basique en tant que solution aqueuse de taurine pour un traitement, de manière à réaliser un recyclage ; ou
la liqueur mère de taurine raffinée est mélangée avec le liquide effluent avant la cristallisation pour un recyclage.

6. Procédé de préparation cyclique à haut rendement du produit fini de taurine colonnaire selon la revendication 3, dans lequel la cristallisation par refroidissement est réalisée au moyen d'une cuve de cristallisation, et un liquide effluent est refroidi à 5-35 °C, de préférence à 15-25 °C.

7. Procédé de préparation cyclique à haut rendement du produit fini de taurine colonnaire selon la revendication 1, dans lequel une concentration massique-volumique de la solution aqueuse de taurine est de 5 %-25 %, de préférence de 10 %-20 %.

8. Procédé de préparation cyclique à haut rendement du produit fini de taurine colonnaire selon la revendication 1, dans lequel la résine basique est une résine faiblement basique, une résine fortement basique, ou une combinaison des deux.

9. Procédé de préparation cyclique à haut rendement du produit fini de taurine colonnaire selon la revendication 1, dans lequel un débit permettant à la solution aqueuse de taurine de traverser la résine basique est de 0,25-25 BV/h, de préférence de 5-15 BV/h.

10. Procédé de préparation cyclique à haut rendement du produit fini de taurine colonnaire selon la revendication 4, dans lequel la base utilisée pour la régénération de la résine basique est un hydroxyde de métal alcalin, un carbonate de métal alcalin, une solution d'ammoniac, ou une combinaison de ceux-ci, dans lequel la base est de préférence de l'hydroxyde de sodium, une solution d'ammoniac, ou une combinaison des deux.

11. Procédé de préparation cyclique à haut rendement du produit fini de taurine colonnaire selon la revendication 4, dans lequel un iséthionate de métal alcalin et un dérivé d'acide iséthionique dans un liquide effluent obtenu après la régénération de la résine basique sont recyclés vers une réaction d'ammonolyse.

12. Procédé de préparation cyclique à haut rendement du produit fini de taurine colonnaire selon la revendication 1, dans lequel le liquide effluent surveillé comme étant qualifié est concentré jusqu'à un rapport massique-volumique de 25 %-40 %, de préférence jusqu'à un rapport massique-volumique de 30 %-35 %, puis cristallisé.

13. Procédé de préparation cyclique à haut rendement du produit fini de taurine colonnaire selon la revendication 3, dans lequel la filtration est réalisée au moyen d'une membrane organique de nanofiltration, d'une membrane céramique de nanofiltration, d'une membrane de nanofiltration en SiC, d'un filtre métallique microporeux ou d'un filtre microporeux en PE, de préférence au moyen de la membrane de nanofiltration en SiC.

14. Procédé de préparation cyclique à haut rendement du produit fini de taurine colonnaire selon la revendication 3, dans lequel la cristallisation est réalisée dans une cuve de cristallisation à une température qui est contrôlée pour être de 60-80 °C, de préférence de 65-75 °C ; et
le processus de refroidissement est le suivant : une température initiale est maintenue à 65-75 °C pendant 0,5 h-1,0 h, et le refroidissement est réalisé à une vitesse de refroidissement de 1 °C-20 °C/h.

15. Procédé de préparation cyclique à haut rendement du produit fini de taurine colonnaire selon la revendication 3 ou 5, dans lequel la séparation solide-liquide est réalisée au moyen d'une centrifugeuse à plateau, d'une centrifugeuse suspendue par le haut, d'une centrifugeuse horizontale à vis, d'un filtre ou d'un filtre-presse à plaques et cadres.

16. Procédé de préparation cyclique à haut rendement du produit fini de taurine colonnaire selon la revendication 1, dans lequel la solution aqueuse de produit brut de taurine est préparée à une température de 0-100 °C, de préférence de 35-80 °C.

17. Procédé de préparation cyclique à haut rendement du produit fini de taurine colonnaire selon la revendication 1, dans lequel, si la teneur du radical iséthionate est supérieure à 0,005 %, le liquide effluent est non qualifié ; et, de préférence, si la teneur du radical iséthionate est supérieure à 0,001 %, le liquide effluent est non qualifié.

18. Procédé de préparation cyclique à haut rendement du produit fini de taurine colonnaire selon la revendication 1, dans lequel l'iséthionate de métal alcalin est de l'iséthionate de sodium de métal alcalin, de l'iséthionate de potassium de métal alcalin ou de l'iséthionate de lithium de métal alcalin.
